# EUROPEAN PATENT SPECIFICATION

(11) **EP 3 371 728 B1**
(45) Date of publication and mention of the grant of the patent: **27.12.2023**
(21) Application number: 16810121.0
(22) Date of filing: 04.11.2016
(51) Int. Cl.: G16H 10/60, G06F 21/62

(54) **SECURING RESOURCES WITH A REPRESENTATIONAL STATE TRANSFER APPLICATION PROGRAM INTERFACE**
RESSOURCENSICHERUNG MIT REPRÄSENTATIVER PROGRAMMSCHNITTSTELLE FÜR ZUSTANDSÜBERMITTLUNGSANWENDUNG
PROTECTION DE RESSOURCES AVEC UNE INTERFACE DE PROGRAMME D'APPLICATION DE TRANSFERT D'ÉTAT REPRÉSENTATIF

(30) Priority: 05.11.2015 US 201514933775
(43) Date of publication of application: 12.09.2018
(73) Proprietor: Boston Scientific Cardiac Diagnostic Technologies, Inc., Rochester, MN 55901 (US)
(72) Inventor: HUTCHINS, James D., Rochester, Minnesota 55901 (US); SMITH, Richard M., Rochester, Minnesota 55901 (US)
(74) Representative: Pfenning, Meinig & Partner mbB
(86) International application number: PCT/US2016/060645
(87) International publication number: WO 2017/079631

(56) References cited:
- US-A1- 2009 328 205
- US-A1- 2011 258 679
- US-A1- 2015 081 866
- US-A1- 2015 120 729
- US-B2- 8 561 088
- US-B2- 9 049 182
- ERIK SUNDVALL ET AL: "Applying representational state transfer (REST) architecture to archetype-based electronic health record systems", BMC MEDICAL INFORMATICS AND DECISION MAKING, BIOMED CENTRAL, LONDON, GB, vol. 13, no. 1, 9 May 2013 (2013-05-09), page 57, XP021153227, ISSN: 1472-6947, DOI: 10.1186/1472-6947-13-57
- Anonymous: "15 minutes Guide to sqlREST", , 3 April 2009 (2009-04-03), XP055252410, Retrieved from the Internet: URL:http://wayback.archive.org/web/2009040 3074907/http://sqlrest.sourceforge.net/5-m inutes-guide.htm [retrieved on 2016-02-23]
- FREDDY PRIYATNA ET AL: "Formalisation and experiences of R2RML-based SPARQL to SQL query translation using morph", WORLD WIDE WEB, INTERNATIONAL WORLD WIDE WEB CONFERENCES STEERING COMMITTEE, REPUBLIC AND CANTON OF GENEVA SWITZERLAND, 7 April 2014 (2014-04-07), pages 479-490, XP058047090, DOI: 10.1145/2566486.2567981 ISBN: 978-1-4503-2744-2
- ALEXANDROS MARINOS ET AL: "HTTP database connector (HDBC)", PROCEEDINGS OF THE 19TH INTERNATIONAL CONFERENCE ON WORLD WIDE WEB, WWW '10, ACM PRESS, NEW YORK, NEW YORK, USA, 26 April 2010 (2010-04-26), pages 1157-1158, XP058297296, DOI: 10.1145/1772690.1772852 ISBN: 978-1-60558-799-8

## Description

### TECHNICAL FIELD

The present disclosure relates to decision-support systems for patient healthcare, and in particular, to data security for systems providing a Representational State Transfer (RESTful) Application Program Interface (API).

### BACKGROUND

Benefits of medical data display systems include rapid presentations and storage of data of monitored health conditions for a patient. At least some of this information may be generated by electronic sensors configured to detect at least one medical condition of a patient and produce an electronic signal based on that medical condition. The electronic signal may be collected over time in a standard metric(s) used in the care of the patient determined for analysis by a medical care provider. Examples of the standard metric may include heart rates and electrocardiogram data. The standard metric can be collected over extended timeframes, for example, in some cases twenty-four hours or more to monitor the health of the patient and to identify abnormalities which may occur with frequencies that may vary on a patient-to-patient basis. The abnormalities may be observable as changes in the at least one standard metric occurring at occurrences that are predictable or still being understood. The abnormalities may be used by the medical care provider to provide long term care to the patient, predict future medical events, or to diagnose medical conditions of the patient.

As the amount of medical data in the form of electrical signals from the patient becomes more readily available and the time demands on the medical caregiver continue to increase, there is a need to provide a highly scalable solution that enables care providers to provide access to the medical information. Moreover, as medical data is frequently incredibly sensitive information, information security is a top concern for any health care system. More generally, outside of the medical space, the amount of data being collected and stored has continued to increase, along with the need to secure such data (particularly personal data). As such, there is a need for a highly scalable solution that ensures the security and integrity of data (*e.g.,* collected medical data).

US9049182B2 discloses techniques for virtual representational state transfer (REST) interfaces, interposing a proxy between a client and a REST service over a network. The proxy performs independent authentication of the client and provides credentials to the client and for the client to authenticate to the REST service using a REST service authentication mechanism. The proxy inspects requests and responses and translates the requests and responses into formats expected by the client and the REST service, enforcing policies.

### BRIEF DESCRIPTION OF THE DRAWINGS

So that the manner in which the above-recited features of the present disclosure can be understood in detail, a more particular description of the disclosure, briefly summarized above, may be had by reference to embodiments, some of which are illustrated in the appended drawings. It is to be noted, however, that the appended drawings illustrate only typical embodiments of this disclosure and are therefore not to be considered limiting of its scope, for the disclosure may admit to other equally effective embodiments.
Figure 1 illustrates an example computing environment, according to one embodiment.
Figure 2 illustrates a parallel processing computing environment, according to one embodiment.
Figure 3 illustrates an event engine for processing received health events, according to one embodiment.
Figure 4 is a block diagram illustrating a system configured with a request processing component, according to one embodiment.
Figure 5 is a flow diagram illustrating a method of processing a request according to a security policy, according to one embodiment.
Figure 6 is a flow diagram illustrating a method of processing a request against a relational database according to a security policy, according to one embodiment.
Figure 7 is a block diagram illustrating a system configured with a request processing component, according to one embodiment.

To facilitate understanding, identical reference numerals have been used, where possible, to designate identical elements that are common to the figures. It is contemplated that elements disclosed in one embodiment may be beneficially utilized on other embodiments without specific recitation.

### DESCRIPTION OF EXAMPLE EMBODIMENTS

### OVERVIEW

The invention is defined by the appended claims.

One embodiment provides a method of enforcing a security policy for requests received by a server application that provides a Representational State Transfer (RESTful) Application Program Interface (API). The method includes receiving, at a server system, a request specifying one of a plurality of predefined actions and a logical resource identifier. The method further includes determining a logical resource uniquely identified by the logical resource identifier. Additionally, the method includes determining user information corresponding to a requestor from which the RESTful API request was received. The method also includes determining a security policy to apply to the request, based on the determined user information and the logical resource. The method also includes processing the received request according to the determined security policy.

An additional embodiment provides a system for carrying out the aforementioned method.

Another embodiment provides a non-transitory computer-readable medium that contains computer program code that, when executed, performs an operation for enforcing a security policy for requests received by a server application that provides a RESTful API. The operation includes providing RESTful API and receiving a Hypertext Transfer Protocol (HTTP) request, formatted in compliance with the RESTful API, and specifying one of a plurality of predefined HTTP actions and a unique logical resource identifier. The operation also includes determining a logical resource corresponding to the unique logical resource identifier. Additionally, the operation includes determining user information corresponding to a requestor from which the HTTP request was received. The operation further includes determining a security policy to apply to the HTTP request, based on the determined user information and the logical resource and determining one or more operations to perform in processing the request, based on a resource type of the logical resource and the predefined HTTP action specified in the HTTP request. In addition, the operation includes processing the received RESTful API request by performing the one or more operations in accordance with the determined security policy.

### EXAMPLE EMBODIMENTS

Network aware devices provide a variety of opportunities for a care provider (*e.g.,* a physician, nurse, technician, *etc*.) to improve patient care. An event manager can use the data provided by network aware devices or an "internet of things" (IoT) device to identify health events that range from identifying critical health care issues such as cardiac or respiratory emergencies to maintenance events where the network aware device fails, *e.g.,* because a battery is low or a wire is disconnected. To process health related events, an event manager may process events using a collection of defined paths.

In one embodiment, a group of servers each host an event engine with a respective set of interconnected tasks and queues that form a workflow. The group of servers may include a load balancer which routes the biometric data measured by the loT devices to one of the servers for processing. Because the biometric data is processed using different tasks, the event engines can process multiple units of biometric data simultaneously. Stated differently, the event engines process units of biometric data to identify occurrences of the health events, using a series of operations in the workflow where each operation (or task) can process a respective unit of biometric data in parallel.

In one embodiment, the event engines classify health events received from the at least one sensor devices which may be worn by the patient. For example, a device may have classified the health event as one type of event. However, the workflows in the event engines may process the biometric data associated with the event to confirm the initial classification or reclassify or reprioritize the health event as a different type of event. Alternatively, the sensor device may send biometric data to the servers which then use one or more thresholds or rules to identify health events which are then processed by the workflows in the event engines. The workflows determine actions to take when detecting occurrences of health events, such as notifying a care provider, suppressing or ignoring the event, or storing the event in a data repository.

In one embodiment, the workflows process the health events using a priority assigned to the health events. The priority may be assigned based on a severity of the event (*e.g.*, an irregular heart beat versus a heart attack), the type of the health event, a characteristic of the patient whose biometric data generated the health event (*e.g.*, the patient's age or past medical history), and the like. Processing nodes in the workflows may include multiple consumers (*e.g.,* processing threads) for processing different priority health events. For example, a processing node may include ten consumers assigned to process high priority events, seven consumers assigned to process medium priority events, and five consumers assigned to process low priority events. By having more consumers assigned to high priority events, the latency for processing these events in the workflow may be reduced. In another example, the consumers assigned to process the higher priority health events may have more computer resources assigned to them - *e.g.,* additional CPUs or memory - than consumers that process lower priority events in the node.

Designing a software architecture capable of managing patient data for a substantial number of patients is very challenging, particular as such monitoring environments continue to scale in size. However, many centralized monitoring systems with conventional Application Program Interfaces (APIs) are ill-equipped to provide such scalability. As a result, scaling such conventional systems to meet the growing demand and user-base can require excessive computing resources and oftentimes requires developers to make undesirable decisions, inefficient decisions and/or inconsistent decisions (*e.g.,* some services could have different semantics than other services created by a different developer) when modifying the conventional system.

As such, embodiments provide techniques for enforcing a security policy in a server application that provides a Representational State Transfer (RESTful) Application Program Interface (API). For instance, a request processing component can provide a Representational State Transfer (RESTful) Application Program Interface (API) for a server system (*e.g.*, a centralized computing system in a patient care environment). For instance, the RESTful system could leverage Hypertext Transfer Protocol (HTTP) request protocols in its implementation, including the standard command verbs that are included in standard HTTP requests. Such command verbs include, for example, a GET command, a POST command, a PUT command, a DELETE command, a PATCH command and so on. However, such an example is provided for illustrative purposes only, and while HTTP formatting is suitable for providing a RESTful API, more generally RESTful APIs can be built on any suitable message format.

Generally, the RESTful API request also specifies a unique resource identifier (URI) that identifies a resource that is the subject of the corresponding command. For example, a RESTful API request could specify a URI of "/patients/john" to uniquely identify a patient with a name value of "John" within the database table "patients," within a database. Upon receiving such a request, the request processing component could be configured to determine an identity of the requesting entity and could execute operations corresponding to the specified action, upon determining that the requesting entity is authorized to perform the requested action on the specified resource. For instance, upon determining that the specified action is a GET action and the requesting entity is authorized to perform such an action against the specified logical resource, the request processing component could execute a query against a database to retrieve one or more data values corresponding to the specified logical resource. As another example, the request could specify a PUT action, a logical resource identifier and one or more data values, and upon determining that the requesting entity is authorized to perform such an action against the resource identified by the logical resource identifier, the request processing component could write the specified one or more data values to a database entity corresponding to the specified logical resource identifier. Of course, such examples are for illustrative purposes only, and more generally any actions and URI can be used, consistent with the functionality described herein.

In determining whether the requesting entity is authorized to perform the requested action, the request processing component can use a hierarchical security model to evaluate the request. For example, consider the URI "/patients/john/devices". The request processing component could evaluate a first request specifying the URI and could determine that the requesting entity is authorized to access "john" and other objects under "john" in the hierarchy, but the requesting entity is not allowed to access "devices" that john has assigned. Thus, in such an example, the requesting entity may not be authorized to access the resource "/patients/john/devices", but could access (*e.g.,* in subsequent requests) the URI "/patients/john/careplans".

Additionally, the request processing component can process requests in accordance with an appropriate security policy for the request. As an example, the request processing component could determine an identity of the requestor (*e.g.,* a current user who is successfully authenticated on a client device) and could enforce role-level security for the request, based on a role(s) assigned to the determined user information. As an example, particular logical resources may only be accessed by users assigned certain roles (*e.g.,* physicians), and the request processing component could verify that the user has the required role to access the specified URI. Additionally, the request processing component can provide other security services when processing the request, such as relationship security (*e.g.,* only processing logical resources that the requesting entity has an existing relationship with) and through the use of predicate expressions to filter response data or perform validation operations on passed data. Doing so provides a highly scalable system in which logical resources can be secured on the centralized server, rather than relying on client applications to perform the appropriate security validation.

### Patient Care Environment

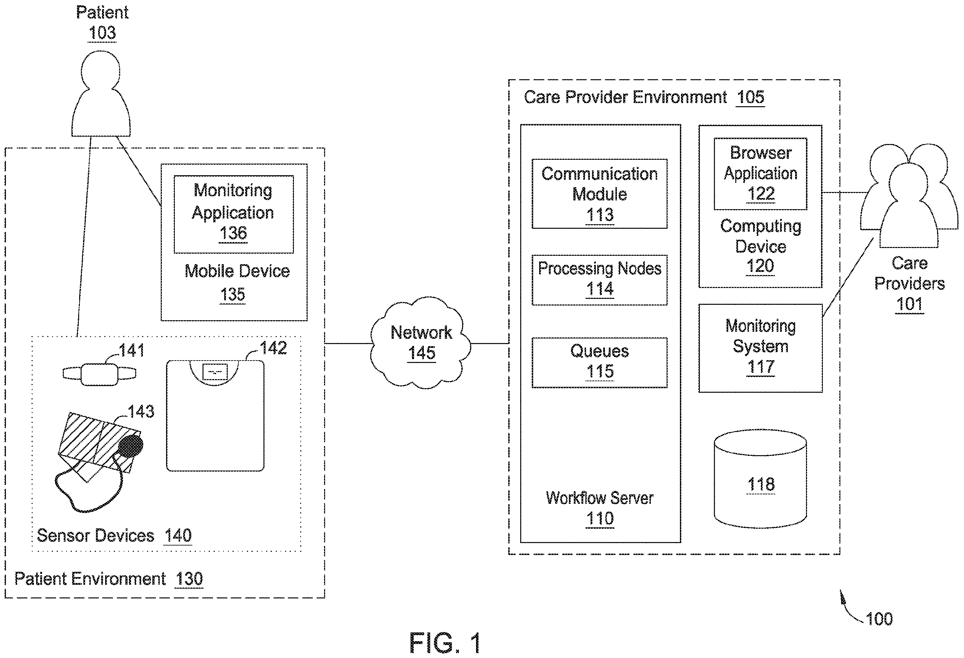

Figure 1 illustrates an example computing environment 100, according to one embodiment. As shown, the computing environment 100 may include a care provider environment 105 and a patient environment 130, each connected to one another via a network 145. The care provider environment 105 and the patient environment 130 allow a care provider 101 (*e.g.,* a technician, nurse, physician, *etc*.) to monitor biometric data generated by the patient 103.

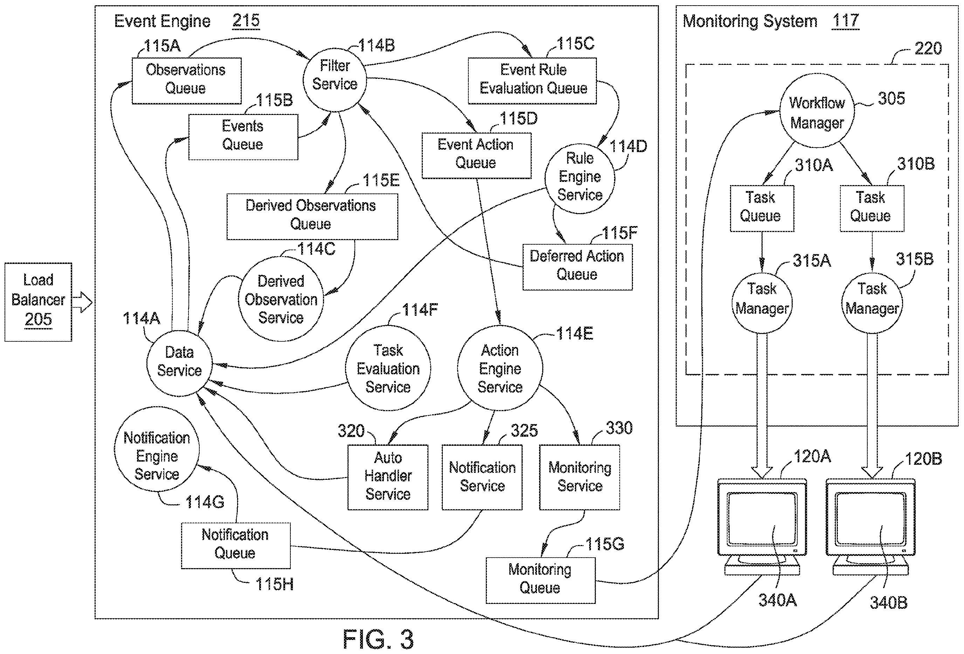

The care provider environment 105 includes a workflow server 110, a computing device 120, monitoring system 117 and data repository 118. Each of the workflow server 110, the computing device 120, and the monitoring system 117 may be a physical computing system that includes one or more computing devices 120A, 120B (Figure 3) or a virtual computer instance (*e.g.,* executing in a cloud computing platform). A care provider 101 may use the computing device 120 to access (*e.g.*, via a browser application 122, a native application on device 120, *etc*.) a user interface (UI) hosted by the monitoring system 117.

Of note, although shown as a single entity, the data repository 118 can represent multiple, separate data stores (*e.g.*, relational databases). Moreover, these data stores can span multiple computing nodes. To this end, the separate data stores could be made to function as a single data store (*e.g.,* through data replication techniques and through the use of load balancers). As such, the data repository 118 is representative of any sort of data store on any number of computing systems, consistent with the functionality described herein.

Additionally, although not shown, the data repository 118 may store data from and/or service requests from various other entities, such as third party applications, partners and affiliates, electronic medical record systems, external monitoring devices and products, analytics engines, data consolidator applications and so on. More generally, it is contemplated that the data repository 118 and, more generally, other elements within the care provider environment 105, can interact with any number of different data originators and receipts, consistent with the functionality described herein. As such, the computing environment 100 is provided merely for illustrative purposes only and without limitation.

The workflow server 110 includes applications and data executed to identify and handle health events corresponding to the patient 103. As shown, workflow server 110 includes a communication module 113, processing nodes 114, and queues 115. In one embodiment, the processing nodes 114 are software code or applications that perform a predetermined task or action on received data (*e.g.*, health events). The workflow server 110 evaluates data received from the patient environment 130 using a set of interconnected processing nodes 114 and the queues 115 which form a workflow. As the biometric data or health events are received from the patient environment 130, the workflow may classify (or reclassify) the data to identify a type of the health event - *e.g.,* presentation or notification to patient/care provider, suppression, classification, aggregation, computation, prioritization/triage, and the like. For example, different types of data received from the patient environment 130 may trigger different types of health events - *e.g*., an irregular heartbeat may trigger a cardiac event, while a signal indicated an electrode has become detached triggers a maintenance event. In one embodiment, at least one sensor device 140 within the patient environment 130 or a monitoring application 136 installed as part of a mobile device 135 within the patient environment 130 may have performed an initial classification of the data or health events. Nonetheless, the workflow server 110 may evaluate the biometric data (or maintenance data) to confirm that this initial classification was correct.

Each type of health event may take a different path through the workflow. That is, different health events may traverse the processing nodes 114 and the queues 115 using different paths. For example, a cardiac event may be evaluated using different processing nodes 114 in the server 110 than a maintenance event. Furthermore, paths through the workflow for the same health event may differ based on a variety of factors such as the severity of the health event, age of the patient 103, other symptoms exhibited by the patient 103, medication taken by the patient 103, and the like. For example, a high priority cardiac event may skip one or more of the processing nodes 114 or the queues 115 and be immediately displayed to the care provider 101 using the monitoring system 117.

The communication module 113 permits the workflow server 110 to receive the data from the patient environment 130 and transmit data to the care providers 101. The communication module 113 may receive data from the at least one sensor device 140 which is used to identify a health event and a corresponding path through interconnected ones of the processing nodes 114 and the queues 115. The communication module 113 helps the care providers 101 complete the workflow by use of the monitoring system 117 and the computing device 120. Moreover, in addition to receiving the data from the patient environment 130, the communication module 113 may enable the workflow server 110 to transmit requests or instructions to the patient environment 130 such as asking the patient 103 if she has any symptoms or instructing the patient 103 to reattach a disconnected electrode (not shown) of the at least one sensor device 140.

In one embodiment, a path used by a health event to traverse the workflow server 110 may include processing nodes 114 that process the health event without user intervention as well as the processing nodes 114 that require input from the care providers 101. For example, one of the processing nodes 114 may filter or screen a health event to determine what queue to place the event, compare the event to one or more rules to determine an action to perform, or store the event. Alternatively, others of the processing nodes 114 may require the care provider 101 to perform an action or provide instructions. For example, the monitoring system 117 may generate a user interface (Ul) for a health event which is then displayed to the care provider 101 by the browser application 122. Once the care provider 101 performs an action (*e.g.*, confirms the classification of the event or agrees with an action suggested by the workflow server 110), the remaining operations of the workflow are performed - *e.g*., send a notification to the patient 103, log the event in the history of the patient 103, route the event to a different one of the care providers 101, reclassify the health event (if the care provider 101 indicated the initial classification was incorrect), or prioritize or triage the health event.

With continued reference to Figure 1, the patient environment 130 includes the mobile device 135 and the at least one sensor device 140. The mobile device 135 includes the monitoring application 136 which permits communication between the at least one sensor device 140 and the care provider environment 105 via the network 145. The monitoring application 136 may configure the at least one sensor device 140 (*e.g.,* loT devices) to monitor biometric data of the one or more patient 103 as specified by a care plan. For example, the monitoring application 136 could configure logic on a heart rate monitoring device worn by the patient to monitor the patient's heart rate. In turn, the monitoring application 136 can send the heart rate data to the workflow server 110 which determines if a heath event is triggered, and if so, executes a workflow to process the event as described above. In another embodiment, the heart rate monitoring device, upon detecting that a threshold condition has been satisfied, could generate and transmit a health event to the mobile device 135, which in turn transmits the health event to the workflow server 110 for processing. However, in other embodiments, some of the tasks performed by the workflow server 110 may be performed by the mobile device 135. That is, the workflow may include tasks performed by the mobile device 135 or the at least one sensor device 140 as well as tasks performed by the workflow server 110.

In one embodiment, the monitoring application 136 receives environmental data from the at least one sensor device 140. Generally, the environmental data informs the monitoring application 136 of environmental conditions in an area proximate to the at least one sensor device 140 and the user - *e.g.,* a room in which the user is located. For example, the at least one sensor device 140 may detect an air quality or pollen count for the patient 103 having a respiratory ailment. In another example, the at least one sensor device 140 may track the user's movements or actions in an environment such as how many times at night the patient 103 goes to the bathroom or if the patient 103 is tossing and turning at night. This environmental data can then be used by the monitoring application 136 by itself, or in combination with the biometric data, to trigger health events which are processed by the workflow server 110.

In one embodiment, the monitoring application 136 may use an output device (*e.g.*, a display or audio system) on the mobile device 135 to provide information to the patient 103. For example, when executing a workflow, one of the processing nodes 114 may ask the patient 103 if she is experiencing any symptoms. To obtain feedback from the patient 103, the monitoring application 136 may display a user interface (UI) on the mobile device 135 which permits the patient 103 to list symptoms. Moreover, the monitoring application 136 may also display general information related to a care plan or the at least one sensor device 140 such as the patient's heart rate or weight, status of the at least one sensor device 140, *etc.*

In one embodiment, the at least one sensor device 140 interacts with the monitoring application 136 and assists the patient 103 in reporting patient vitals and other information to the care provider environment 105. As shown, the at least one sensor device 140 may include a body sensor 141, a weighing scale 142, and a blood pressure cuff 143. Each of the at least one sensor device 140 may capture different vitals of the patient 103. For example, when applied to a body of patient 103, the body sensor 141 captures biometric data (*e.g*., heart rate, ECG data, *etc*.) in real-time. In addition, each of the at least one sensor device 140 may be configured to transmit body-related metrics electronically to the monitoring application 136 on the mobile device 135. In turn, the monitoring application 136 sends the captured metrics to the workflow server 110 which can be used to trigger health events which are processed using the processing nodes 114 and the queues 115.

In one embodiment, upon detecting an observation threshold has been reached, the at least one sensor device 140 performs an initial classification of the health event. In a particular embodiment, the mobile device 135 is configured to perform the initial classification of the health event. For example, the body sensor 141, upon detecting that ECG data collected from the patient 103 indicates an erratic heart behavior, could classify the health event as a cardiac event. This initial classification of the health event, along with the relevant ECG data (*e.g.,* ECG data including a predetermined length of time before and after the event), could be transmitted to the mobile device 135 (*e.g.,* over a Bluetooth^{®} communications link) and the monitoring application 136 subsequently forwards the ECG data and the health event data on to the workflow server 110 over the network 145 (*e.g.*, the Internet). Alternatively, instead of classifying the data, the monitoring application 136 may forward the raw, unprocessed sensor data to the workflow server 110 which uses one of the processing nodes 114 to identify and classify health events which are then processed in the workflow server 110.

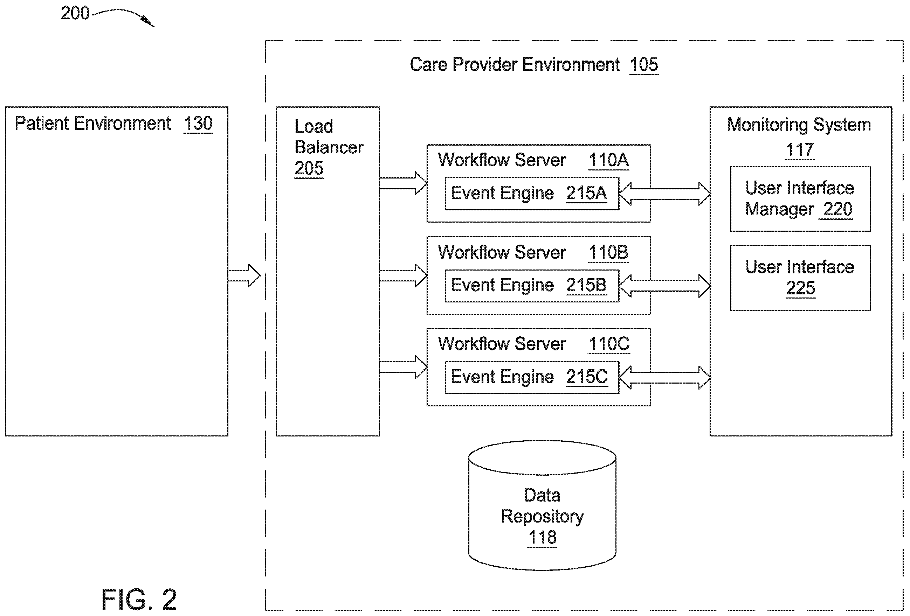

Figure 2 illustrates a parallel processing computing environment 200, according to one embodiment. As shown, the patient environment 130 transmits biometric data and/or health events to the care provider environment 105 which includes a load balancer 205. The workflow servers 110A-110C each include a respective one of the event engines 215A-215C. Although not shown, each of the event engines 215A-215C includes a plurality of interconnected processing nodes and queues that form a workflow for processing health events as discussed above. In one embodiment, the event engines 215A-215C each includes the same processing nodes and queues arranged in the same manner such that any one of the event engines 215A-215C can process the different health events generated by the at least one sensor device 140 - *i.e.,* any one of the event engines 215A-215C can process a cardiac event, respiratory event, maintenance event, *etc.* Based on current workload, the load balancer 205 transmits received data or heath events to one of the workflow servers 110A-110C for processing. For example, the load balancer 205 may assign the received health events in a round robin manner or by monitoring each respective central processing unit (CPU) or memory usage of the workflow servers 110A-110C.

Alternatively, the event engines 215A-215C may have different processing nodes and queues (or a different arrangement of the nodes and queues) such that the event engines 215A-215C are configured to process different event types. For example, the event engines 215A, 215B may have workflows that process cardiac events (and have the same processing nodes and queues), while the workflow in the event engine 215C processes respiratory events. The load balancer 205 may determine which of the event engines 215A-215C should receive the health event using the initial classification provided by the patient environment 130 or based on which of the at least one sensor device 140 measured the biometric data.

Regardless whether the event engines 215A-215C have the same arrangement or different arrangements, compute resources can easily be adjusted in response to varying workloads. For example, if additional sensor devices (*e.g.,* sensor devices 140) are added to the patient environment 130, a system administrator can add additional ones of the workflow servers 110A-110C to process an increased number of received health events. The reverse is also true. If the number of health events decreases, the administrator may remove one or more of the workflow servers 110A-110C. For example, if the event engines 215A, 215B both process cardiac events but the number of cardiac events has decreased, the system administrator may remove one of the workflow servers 110A, 110B. As another example, a load balancer component could monitor the usage of computational resources by the workflow servers 110A-110C and could scale the number of servers up or down, based on the computational resource usage.

With continued reference to Figure 2, the monitoring system 117 includes a user interface manager 220 (Ul manager) and a user interface 225 (UI). As discussed above, the processing nodes 114 may require input from the care provider 101 (Figure 1) in order to route the health events through the event engines 215A-215C. To do so, the event engines 215A-215C transmit requests to the Ul manager 220 which generates the Ul 225 which can be displayed to the care provider 101. For example, the Ul manager 220 may generate the UI 225 that includes an electrocardiogram (ECG) chart corresponding to a cardiac event. Further, the Ul 225 may include I/O features (*e.g.,* buttons or pull down menus) that the care provider can use to provide input or instructions to one of the event engines 215A-215C. For example, the care provider may instruct the one of the event engines 215A-215C to store the cardiac event in the data repository 118, send the cardiac event to one of the queues 115 (Figure 1) that is monitored by another care provider (*e.g.*, to get a second opinion), or forward the cardiac event to the care provider 101 of the patient 103. Thus, the monitoring system 117 permits the workflow servers 110 to output information to the care provider 101 as well as receive instructions from the care provider 101.

The event engines 215A-215C may store data in and retrieve data from the data repository 118. For example, the event engines 215 may maintain a patient history by storing all the received health events (or selected health events) derived based on monitoring a patient's vitals in the repository 118. Further, the event engines 215A-215C may use the data stored in the data repository 118 to process the health events. For example, if one of the event engines 215A-215C receives biometric data indicating the current weight of the patient 103, then the one of the event engines 215A-215C can retrieve past weight measurements for the patient 103 from the data repository 118 and derive a trend graph detailing how the weight of the patient 103 has changed over time. For instance, the patient's current weight may not be enough to trigger a health event, but the patient's derived weight change over a period of time may trigger a health event. As discussed below, these derived trends may be used to generate a derived observation (or other event(s)).

In one embodiment, the event engines 215A-215C prioritize health events, which, in turn, determines how quickly the health events are processed by the workflows in the event engines 215A-215C or what processing nodes and queues are used to process the health events. As discussed above, the health events may be prioritized based on a severity of the health event, the type of the health event, a characteristic of the patient 103 whose biometric data generated the health event, and the like. Additionally, the health events could be prioritized based on additional criteria, such as an institutional policy, a care plan-level policy, a patient-level policy, another policy or some combination of the above.

Figure 3 illustrates one of the event engines 215A-215C, depicted generically as an event engine 215, including a workflow for processing health events, according to one embodiment. As described above, a health event or biometric data received from the at least one sensor device 140 (Figure 1) is forwarded from the load balancer 205 to the event engine 215. Specifically, a data service node 114A in the workflow receives the forwarded information from the load balancer 205. If the load balancer 205 forwards a health event, the data service node 114A classifies the health event based on type (*e.g.,* a cardiac, respiratory, or maintenance event). In some cases, the health event was classified before being received by the data service node 114A. Nonetheless, the data service node 114A may review the data associated with the health event such as ECG data, breathing rate, blood pressure, *etc.* using more compute intensive techniques to determine whether the initial classification was correct. In another example, the data service node 114A may provide a more detailed classification of the health event than the initial classification. For example, the at least one sensor device 140 may have generated the health event because it detected an irregular heartbeat. However, the data service node 114A may evaluate the heartbeat and classify the health event as a specific cardiac health event - *e.g.,* a ventricular trigeminy event or an atrioventricular block event. The data service node 114A may save the classification of the health event which is used by downstream nodes and queues to process the health event.

Instead of receiving a health event, the data service node 114A may receive raw data or observations from the patient environment 130. That is, the raw data or observations may not have been evaluated by the at least one sensor device worn by the patient to determine if this data triggers a health event. For example, observation data from a sensor includes blood pressure measurements, weight measurements, ECG data, and the like. As discussed below, the event engine 215 evaluates these observations and can trigger health events which are then processed in the engine 215.

With continued reference to Figure 3, the data service node 114A forwards the observations to the observation queue 115A and the health events to the events queue 115B. A filter node 114B pulls the observations and health events stored in the queues 115A and 115B. This node 114B serves as a gatekeeper that determines where the health events and observations are routed for further processing. When evaluating observations, the filter node 114B may determine whether to ignore (*i.e.,* drop) the observations or forward the observations to a derived observation queue 115E. For example, observations such as low battery signals, start signals indicating the at least one sensor device 140 has started collecting biometric data, or stop signals indicating the at least one sensor device 140 has stopped may be ignored by the filter service node 114B. In contrast, the node 114B may forward observations such as weight measurements, blood pressure measurements, ECG data, and the like to the derived observation queue 115E. In this manner, the filter service node 114B screens the incoming observations to determine whether they should be processed further such as checking for triggering health events.

Observations forwarded by the filter service node 114B are then processed by a derived observation service node 114C. This derived observation service node 114C uses received observations in conjunction with previously received observations to create new observations or to generate a new health event. Stated differently, the derived observation service node 114C may aggregate previously received observations with the currently received observations to compute statistics, trends, trigger health events, and the like. Although not shown, the derived observation service node 114C may be communicatively coupled to the data repository which stores past observations. For example, if the currently received observation is a weight measurement, the derived observation service node 114C may evaluate this measurement with previous weight measurements to determine a weight change for the patient over a defined period of time. This weight change may trigger a health event which is then forwarded to the data service node 114A for further processing. Even if a health event is not triggered, the derived observation service node 114C may store a derived observation (*e.g.*, a weight change, average blood pressure, heart rate trends, *etc*.) in the data repository so that this data is available when further observations for the patient are received by the event engine 215 (or other event engines 215).

In one embodiment, health events may be processed by the derived observation service node 114C. For example, one of the at least one sensor device 140 may trigger a health event upon determining a patient's average blood pressure for a day exceeds a threshold. The filter service node 114B may forward this health event to the derived observation service node 114C which then may use past blood pressure measurements for that patient 103 to derive a weekly or monthly average blood pressure for the patient 103, or a blood pressure trend graph. Based on this derived observation, the derived observation service node 114C may generate a new health event or decide to drop the health event if the derived observation does not satisfy a corresponding condition.

Further, the filter service node 114B also includes logic for determining whether received health events should be dropped, forwarded to an event action queue 115D, or forwarded to the event rule evaluation queue 115C. For example, a system administrator may determine that some health events are not relevant for certain patients. The logic in the filter service node 114B may identify and drop these health events to prevent them from propagating through the rest of the event engine 215. For instance, a patient may have a heart murmur that constantly results in a sensor device triggering a health event. Rather than continually processing these health events, the care provider 101 can instruct the filter service node 114B to screen out (or suppress) these health events from the patient 103.

If a received health event has a corresponding action or actions, the filter service nodes 114B forwards the health event to the event action queue 115D. However, if the action for a health event has not yet been identified, the filter service node 114B forwards the health event to the event rule evaluation queue 115C. A rule engine service node 114D pulls the health events from the queue 115C and evaluates the health event using one or more rules. An example of the one or more rules includes determining whether daily weight change and average blood pressure exceed respective thresholds. Based on this evaluation, the rule engine service node 114D may determine what action the event engine 215 should perform - *e.g*., suppress/ignore the event, auto handle the event, display the event to a care provider, or delay processing the event. Once the action is determined, the rule engine service node 114D generates and forwards a new health event that includes the corresponding action to the data service node 114A. Now that the corresponding action is known, once the new health event reaches the filter service node 114B, it forwards the health event to the event action queue 115D rather than the event rule evaluation queue 115C.

With continued reference to Figure 3, the rule engine service node 114D may delay processing the health event by forwarding the health event to a deferred action queue 115F. That is, if all of the rules have not yet been evaluated and further evaluation is required before triggering the event action, then the health event may be placed in the deferred action queue 115F. For example, the rule may trigger a cardiac event but the monitoring system 117 must first check to determine if that health event is suppressed for the patient 103 before taking the corresponding action. As shown, the health events stored in the deferred action queue 115F are then retrieved by the filter service node 114B and can be reintroduced into the event rule valuation queue 115C at a later time - *i.e.,* when all the rules have been evaluated.

Once a corresponding action for a health event is known and the health event is stored in the event action queue 115D, an action engine service node 114E routes the health event to the appropriate action service - *i.e*., auto handler service 315, notification service 320, or monitoring service 325. The auto handler service 315 may perform actions that do not require supervision or input by a care provider - *e.g.,* stores the health event in the data repository. As another example, the auto handler service 320 may assign a priority or severity to the health event before the event is reintroduced into the workflow with the new priority. The auto handler service 320 may also generate a new health event when, for example, a health event shows a cardiac event but the data quality is low. In response, the auto handler service 320 may introduce a maintenance event for checking the sensor connection/electrodes.

With continued reference to Figure 3, the event engine 215 uses notification service 325 to send information to the patient 103, the care provider 101, or the at least one sensor device 140 regarding the health event. The notification service 325 may include different communication channels or techniques for communicating with the patient such as email, chat, SMS messages, *etc.* Although Figure 3 illustrates only one notification queue 115H and notification engine service node 114G for handling requests, the event engine 215 may have different queues and notification nodes for the different communication techniques. For example, if a maintenance event is triggered when an electrode is unplugged from the at least one sensor device 140, then the notification service 325 may transmit an email to the patient's mobile device instructing the patient to plug in the electrode. Alternatively, if a respiratory event is triggered because of an elevated breathing rate, the notification service may send an SMS message to the patient 103 asking her if she is currently performing a physical activity.

The event engine 215 also includes a task evaluation service node 114F. Unlike the other nodes and queues in event engine 215 which process or store observation data or health events received from the patient environment, the task evaluation service node 114F determines whether to trigger a health event based on a care protocol or care plan. In one embodiment, the node 114F triggers a health event when the patient does not follow the care protocol or plan. For example, the care protocol may ask that the patient wear one of the at least one sensor device 140 for certain amount of time during the day or take weight measurements each day. By monitoring the observation and health events received by the event engine 215, the task evaluation service node 114F determines whether the patient 103 has complied with the care protocol. If not, the task evaluation service node 114F triggers a health event with a corresponding action for the event engine 215 to perform such as sending a notification to the patient 103 using notification service 325 or informing a care provider using a monitoring service 330.

The monitoring service 330 communicatively couples the event engine 215 to the monitoring system 117. When input from a care provider regarding a health event is desired, the monitoring service 330 forwards the health event to a monitoring queue 115G. The Ul manager 220 in the monitoring system 117 includes a workflow manager node 305 that pulls health events from the monitoring queue 115G and assigns them to either a task queue 310A or a task queue 310B. The UI manager 220 also includes task manager node 315A and task manager node 315B which generate Uls for the health events. These Uls are then displayed to care providers 101 via the at least one computing device 120A and 120B. Further, the task manager nodes 315A, 315B may place the biometric or maintenance data associated with the health events in the Uls. For example, a Ul for a cardiac event may display an ECG graph and a baseline chart, while a Ul for respiratory event displays a breathing rate and oxygen levels in the blood. In this manner, the Ul manager 220 can generate a customized UI for the different health events.

The at least one computing device 120A, 120B may transmit information to the data service node 114A of the event engine 215 which can be used to generate new health events or update current health events. For example, the care provider 101 may instruct the event engine 215 to take a certain action such as forwarding the health event to a different care provider to get a second opinion, reclassifying the health event, suppressing or ignoring the health event, notifying a health care provider, and the like. Based on the care provider's input, the event engine 215 again routes the health event through the nodes 114 and queues 115.

The user interfaces 340A, 340B (Uls) respectively output on the computing devices 120A, 120B can be generated to enable the care provider 101 to easily recognize correlations between health events (*e.g.,* ECG events) and a monitored health metric (*e.g.*, a patient's average heart rate over a period of time). The ability to easily identify groups of the health events as well as understand the time that the health event occurred can better enable the care provider 101 to accurately diagnose a condition of the heart of a patient 103 and thereby provide a more effective treatment.

### Securable Resources

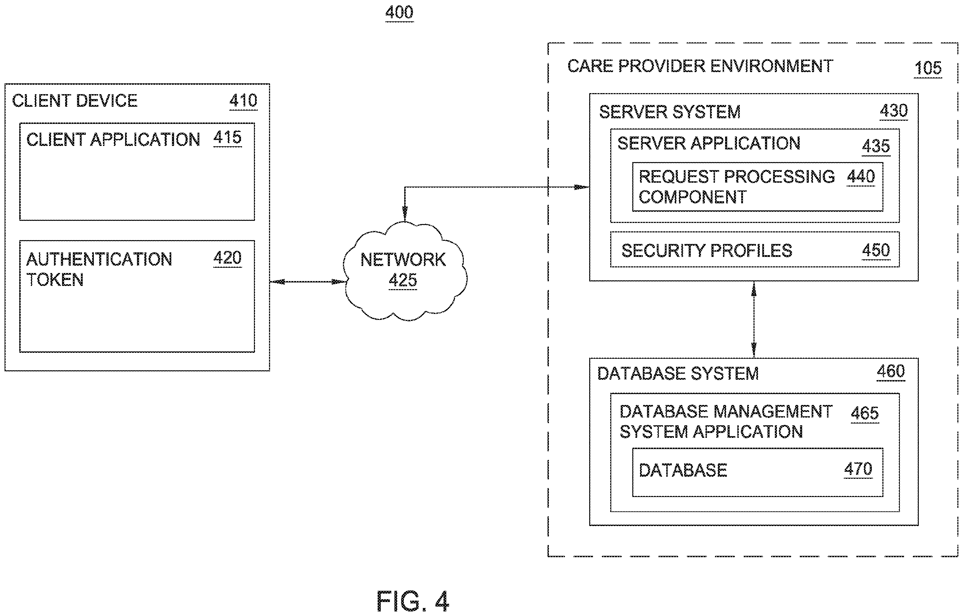

Figure 4 is a block diagram illustrating a system configured with a request processing component, according to one embodiment. As will be discussed below, such an architecture enables security to be controlled and applied at the server level, rather than relying on various calling applications (*e.g.,* applications on the client device 410) to properly apply the defined security restraints. As shown, the system 400 includes a client device 410 and the care provider environment 105, interconnected via a network 425. The client device 410 includes a client application 415 and an authentication token 420. Generally, the client device 410 represents any computer system capable of hosting the client application 415 (*e.g.*, a computer system within a monitoring center, a physician's computer system, a patient's computer system or mobile device, *etc*.). For example, the client application 415 could be a monitoring application (*e.g.*, where the client device 410 resides within the monitoring system 117).

Generally, the authentication token 420 represents a unique digital signature that is assigned to a user of the client device 410 and can be used in authenticating the user. Although a single authentication token 420 is shown, it is contemplated that any number of authentication tokens 420 can reside on (or be accessible by) the client device 410. Moreover, while the authentication token 420 is shown as residing within the client device 410, more generally the authentication token 420 can reside on any storage device that is accessible by the client device 410 (*e.g.*, a flash memory device communicatively connected to the client device 410). Additionally, the authentication token 420 can be issued to the client device 410 by an identity server 418. For example, the identity server 418 could issue the authentication token 420 to the client device 410 upon receiving identity information (*e.g.,* login and password information) from the client device 410 that matches preconfigured identity information corresponding to the user sotred on the identity server. The identity server 418 can also perform operations to manage the issued authentication token 420. Such operations may include, without limitation, controlling the expiration of the authentication token 420 and managing the validity of the authentication token 420. Of note, while the identity server 418 is shown as residing on the client device 410 in the system 400, more generally such an identity server 418 reside on any system that is communicatively coupled to the client device 410, *e.g.,* a computing system coupled to the network 425, *etc.*

The care provider environment 105 includes a server system 430 and a database system 460. The server system 430 includes a server application 435 and security profiles 450. Generally, the security profiles 450 specify one or more rules defining how to secure a resource(s) within the database 470. For instance, security profiles 450 could provide rules defining how to secure a particular type of resource (*e.g.*, patient data). Additionally, the security profiles 450 could provide rules defining how to secure resources based on attributes of the resources. As an example, a security profile 450 could define patient data for a particular patient within the database 470 as accessible only by the particular patient or by other users having a predefined relationship with the particular patient (*e.g*., authorized family members, the patient's healthcare providers, *etc*.)*.* The server application 435 includes a request processing component 440. The database system 460 includes a database management system (DBMS) application 465, which in turn includes a database 470. Generally, the DBMS application 465 manages access to the database 470 (*e.g*., processing queries against the database 470).

Generally, the request processing component 440 provides a RESTful API for the server system 430. That is, the request processing component 440 is generally configured to process RESTful API requests received by the server system 430. Generally, a RESTful API refers to a software architecture that is commonly used to build highly scalable and highly available web servers. Typically (although not exclusively), RESTful systems leverage Hypertext Transfer Protocol (HTTP) requests in their implementation, including the standard command verbs that are included in standard HTTP requests. Such command verbs include, for example, a GET command, a POST command, a PUT command, a DELETE command, a PATCH command and so on. A RESTful API request further specifies a unique resource identifier (URI) that identifies a resource that is the subject of the corresponding command. For example, a RESTful API request could specify a URI of "/patients/john" to uniquely identify a patient with a name value of "John" within the database table "patients," within the database 470 managed by the DBMS application 465. Of course, such an example is for illustrative purposes only, and more generally any URI can be used, consistent with the functionality described herein.

The request processing component 440, upon receiving a RESTful API request specifying an action and a logical resource identifier, can process the received request while enforcing an appropriate security policy for the request. For instance, the request processing component 440 could determine a logical resource corresponding to the logical resource identifier and could determine user information corresponding to a requestor from which the RESTful API request was received. As an example, the request processing component 440 could determine an identity of a user who is successfully authenticated (*e.g.,* based on the user submitting particular login and password information, together with the authentication token 420 assigned to the user) in a current session for the client application 415 on the client device 410 from which the RESTful API request was received. The request processing component 440 could then determine a security policy to apply to the RESTful API request, based on the determined user information, the logical resource and the requested operation. For instance, the request processing component 440 could select one or more of the security profiles 450 to apply to the request. The request processing component 440 could process the received RESTful API request according to the determined security policy.

Generally, the request processing component 440 can enforce a number of different types of security policies when processing the request. For instance, the request processing component 440 could enforce role-level security for the request, based on a role(s) assigned to the determined user information. As an example, particular values within the database 470 may only be accessed by users assigned certain roles (*e.g.,* physicians), and the request processing component 440 can verify that the user has the required role to access the specified resource URI.

Additionally, the request processing component 440 can enforce a relationship security policy when processing the request. That is, the request processing component 440 could be configured to allow access to particular values within the database 470, when the requestor has a particular relationship to the requested data. For instance, the request processing component 440 could allow access to patient data for a particular patient stored within the database 470, only to users who share a particular relationship with the particular patient (*e.g.,* the patient himself, the patient's authorized family members, the patient's health care providers, *etc*.).

Furthermore, the request processing component 440 can be configured to apply one or more predicate expressions to a set of results of processing the received request, before returning the results to the client application 415. For example, a predicate expression could limit a request for all user data to include only patient data (*e.g.*, while excluding other data such as provider data and administrator data). In addition, a predicate expression could be used to enforce a constraint on the type of resource (*e.g.* a user) that a caller is allowed to create. As an example, an administrator could be limited to creating only provider-type users, while a provider could be limited to creating only patient profiles.

In a particular embodiment, the request processing component 440 is configured to retrieve the security profiles 450 from the database 470 by submitting one or more queries to the DBMS application 465. For instance, a system administrator could write the rules (*e.g.*, as conditional expressions) to the database 470 and the request processing component 440 could retrieve the rules from the database for use in processing requests. Doing so provides a dynamic and scalable solution in which system administrators can modify the security profiles 450 without having to alter the computer program code of the request processing component 440.

In one embodiment, the request processing component 440 generates a Structured Query Language (SQL) query based on the HTTP action and URI specified within the RESTful API request, as well as the determined security policy, and could submit the SQL query to the DBMS application 465 for execution against the database 470. The request processing component 440 could then return a result (*e.g.,* query results returned by the DBMS application 465, an acknowledgement message, an error message, *etc*.) to the client application 415.

Generally, the request processing component 440 can produce a number of various results when processing requests. For instance, upon determining that the authenticated user submitting the request has sufficient access to perform the specified action against the specified URI, the request processing component 440 could process the request by submitting a query to the DBMS application 465 to be executed against the database 470, and returning the query results to the client application 415. On the other hand, if the request processing component 440 determines that the authenticated user does not have sufficient access, the request processing component 440 could return an error message to the client application 415, indicating that the request was not processed. In a particular embodiment, the request processing component 440 returns a subset of the query results, based on the scope of the request and what the requester is authorized to access. Doing so can prevent the requesting entity from issuing a subsequent database call to request only the portion of the results that the requestor is authorized to access, which in turn would generate additional workload for the request processing component 440 and the database system.

In some instances, where the request processing component 440 determines that the user does not have sufficient access to perform the specified action against the specified resource, the request processing component 440 could submit a limited query to the DBMS application 465 and could return the resulting query results to the client application 415. For instance, for a GET request against a particular URI, the request processing component 440 could generate a query configured to retrieve only the data values for the particular URI that the requesting user has sufficient access to, and could submit the limited query to the DBMS application 465 for execution against the database 470. Alternatively, the request processing component 440 could submit an unrestricted query to the DBMS application 465 to retrieve all values for the specified resource, and the request processing component 440 could filter the query results to include only the values the user is allowed to access, prior to returning the query results to the client application 415.

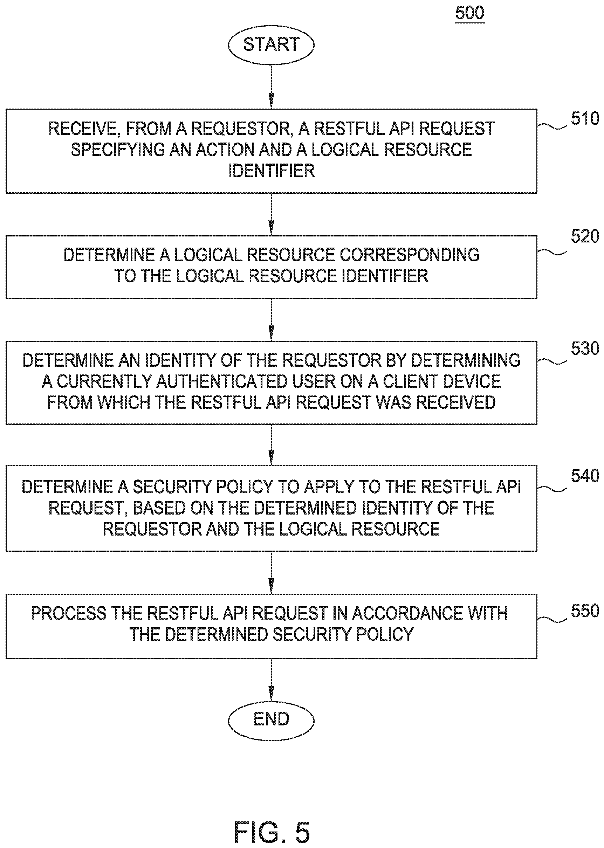

Figure 5 is a flow diagram illustrating a method of processing a request according to a security policy, according to one embodiment. As shown, the method 500 begins at block 510, where the request processing component 440 receives a RESTful API request from a requestor. The request specifies an action and a logical resource that is the subject of the action. The request processing component 440 determines a logical resource corresponding to the logical resource identifier (block 520). As an example, the request processing component 440 could specify an HTTP action (*e.g.*, a GET action, a PUT action, a PATCH action, a DELETE action or a POST action) and could specify a URI that uniquely identifies a particular logical resource (*e.g.,* a logical element within a relational database).

In one embodiment, the request processing component 440 may perform a different operation corresponding to the action specified in the request, depending on a type of the logical resource. For instance, if the request identifies a resource of the database table type and specifies a PATCH action, the request processing component 440 could update a particular value within the database table upon determining that the requestor is authorized to perform the specified action. As another example, if the request identifies a resource of the database value type and specifies a PATCH action, the request processing component 440 could deny the request upon determining that a PATCH action is not a valid action for a database value.

The request processing component 440 also determines an identity and role of the requestor by determining a currently authenticated user on a client device from which the request was received (block 530). For instance, the request processing component 440 could access a session object for the client device to determine the currently authenticated user on the client device. As an example, in order to authenticate the user, the client device could require the user to enter valid login and password information. Additionally, the request processing component 440 could retrieve the identity and role information by extracting such information from a security token assigned to requesting entity.

The method 500 continues, where the request processing component 440 determines a security policy to apply to the received request, based on the requestor's identity and the logical resource specified in the request (block 540). The request processing component 440 then processes the request in accordance with the determined security policy (block 550) and the method 500 ends. As discussed above, the request processing component 440 can be configured to secure the logical resource through a combination of role-level security, relationship-level security and predicate expressions. In other words, the request processing component 440 could provide role-level security based on a role assigned to the requestor.

Additionally, the request processing component 440 could provide relationship-level security for the logical resource. As an example, the security policy could define the role of "physician" as having full access to information on available resources for a particular hospital. As another example, the security policy could define the role of "patient" as having access to only the medical records that are specific to the requestor. Moreover, the request processing component 440 could be configured (*e.g.,* by a system administrator) with one or more predefined predicate expressions which could further filter data and perform expression-based validations when updating the logical resource. As an example, a predicate expression could be defined that further subsets a list of resources using a property of the resource, *e.g*., a request specifying a GET action for a particular logical resource could retrieve values for all users the requestor is authorized to access, and a predicate expression could then be evaluated to further filter the retrieved values to only those values relating to a particular type of user. As another example, a predicate expression could be used to perform validation operations, *e.g.,* enforcing a constraint on the type of resource a caller is allowed to create. Thus, a requesting entity with an "administrator" role may be limited to the creation of "provider" type users, while a requesting entity with the "provider" role may be limited to creating only patient profiles.

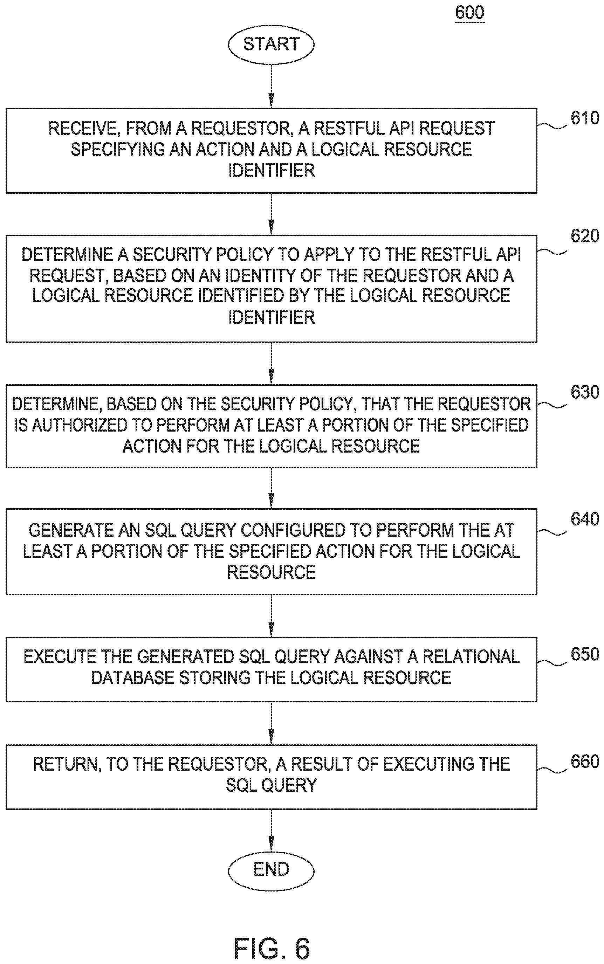

Figure 6 is a flow diagram illustrating a method of processing a request against a relational database according to a security policy, according to one embodiment. As shown, the method 600 begins at block 610, where the request processing component 440 receives, from a requestor, a request specifying an action and a logical resource identifier. The request processing component 440 determines a security policy to apply to the received request, based on an identity and role of the requestor and the logical resource identified by the logical resource identifier (block 620). For example, the request processing component 440 could determine the security policy to apply to the requestor as shown in blocks 520, 530 and 540 of the method 500. Of note, while a particular identity can be assigned several different roles, the authentication token assigned to the requesting entity can be used to determine the specific role to be used when processing the received request.

Returning to the method 600, the request processing component 440 determines, based on the security policy, that the requestor is authorized to perform at least a portion of the specified action for the logical resource (block 630). That is, in some circumstances, the request processing component 440 could determine that, for a request specifying to GET all data values for a particular logical resource (*e.g.,* a particular table of a relational database), the requesting user is authorized to access all of the data values within the specified table (*e.g.,* based on the user's role). As another example, the request processing component 440 could determine that, for another request specifying to GET all data values for the particular logical resource, the requesting user is authorized to access only those data values within the particular logical resource which have a patient identifier that matches the requesting user's patient identifier (*e.g.,* a patient's own medical records), based on a relationship-level security policy.

Upon determining that the requestor is authorized to perform at least a portion of the specified action, the request processing component 440 generates an SQL query configured to perform the at least a portion of the specified action for the logical resource (block 640). The request processing component 440 then facilitates the execution of the generated SQL query against a relational database storing the logical resource identified in the request (block 650). For example, the request processing component 440 could submit the generated SQL query to a DBMS managing the relational database for execution. The request processing component 440 then returns, to the requestor, a result of executing the SQL query (block 660), and the method 600 ends.

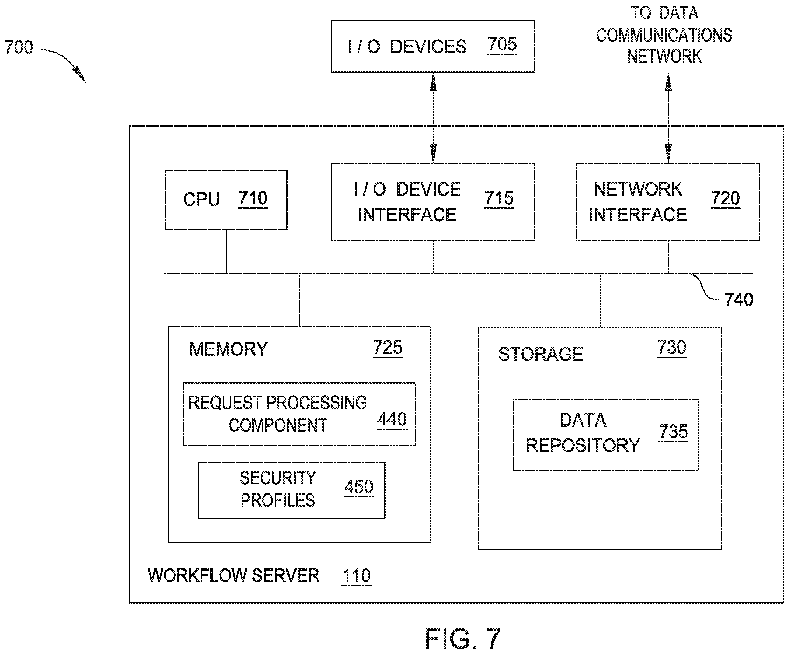

Figure 7 illustrates a computing environment 1100 for processing health events, according to one embodiment. As shown, workflow server 110 includes, without limitation, a central processing unit (CPU) 710, a network interface 720, a memory 725, and storage 730, each connected to a bus 740. The workflow server 110 may also include an I/O device interface 715 connecting I/O devices 705 (*e.g.,* keyboard, display and mouse devices) to the workflow server 110. Further, in context of this disclosure, the computing elements shown in the workflow server 110 may correspond to a physical computing system (*e.g.,* a system in a data center) or may be a virtual computing instance executing within a computing cloud.

Generally, CPU 710 retrieves and executes programming instructions stored in the memory 725 as well as stores and retrieves application data residing in the storage 730. The bus 740 is used to transmit programming instructions and application data between the CPU 710, the I/O devices interface 715, the storage 730, the network interface 720, and memory 725. Note, CPU 710 is included to be representative of a single CPU, multiple CPUs, a single CPU having multiple processing cores, and the like. The memory 725 is generally included to be representative of a random access memory. The storage 730 may be a disk drive storage device. Although shown as a single unit, the storage 730 may be a combination of fixed and/or removable storage devices, such as fixed disc drives, removable memory cards, network attached storage (NAS), or a storage area-network (SAN).

Illustratively, the memory 725 includes the request processing component 440 and security profiles 450, while storage 730 includes a data repository 735. In one embodiment, the data repository 735 represents a relational database managed by a Database Management Server (DBMS) (not shown) on the workflow server 110. Although security profiles 450 are shown as residing within memory 725, more generally the security profiles 450 may reside in other locations on or accessible by the workflow server 110. For example, in one embodiment, the request processing component 440 is configured to access security profiles 450 stored within the data repository 735.

Generally, the request processing component 440 is configured to enforce a security policy specified in the security profiles 450, for a server application (not shown) that provides a RESTful API. For instance, the request processing component 440 could receive a RESTful API request specifying an action and a logical resource identifier. The request processing component 440 could then determine a logical resource corresponding to the logical resource identifier. The request processing component 440 could further determine user information corresponding to a requestor from which the request was received (*e.g.,* a currently authenticated user on a client device from which the request was received). The request processing component 440 could determine a security profile 450 to apply to the request, based on the determined user information, the logical resource, and the request processing component 440 could process the request according to the determined security policy.

In the preceding, reference is made to embodiments presented in this disclosure. The invention is defined in the appended claims.

As will be appreciated by one skilled in the art, the embodiments disclosed herein may be embodied as a system, method or computer program product. Accordingly, aspects may take the form of an entirely hardware embodiment, an entirely software embodiment (including firmware, resident software, micro-code, *etc*.) or an embodiment combining software and hardware aspects that may all generally be referred to herein as a "circuit," "module" or "system." Furthermore, aspects may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium is any tangible medium that can contain, or store a program for use by or in connection with an instruction execution system, apparatus or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, *etc.,* or any suitable combination of the foregoing.

Computer program code for carrying out operations for aspects of the present disclosure may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present disclosure are described below with reference to flowchart illustrations and/or block diagrams of methods, apparatus (systems) and computer program products according to embodiments presented in this disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

The flowchart and block diagrams in the Figures illustrate the architecture, functionality and operation of possible implementations of systems, methods and computer program products according to various embodiments. In this regard, each block in the flowchart or block diagrams may represent a module, segment or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

In view of the foregoing, the scope of the present disclosure is determined by the claims that follow.

## Claims

1. A method, comprising:
receiving, at a server system (430), a request specifying one of a plurality of predefined actions and a logical resource identifier;
determining a logical resource uniquely identified by the logical resource identifier;
determining user information corresponding to a requestor from which the RESTful API request was received;
determining a security policy to apply to the request, based on the determined user information and the logical resource;
enforcing role level security for the request, based on role(s) assigned to the determined user information,
particular logical resources may only be accessed by users assigned to certain roles, one role being a physician, and
care plans and devices of a patient are configured as different logical resources, and different logical resources can only be accessed by users assigned certain roles,
processing the received request according to the determined security policy;
processing the received request according to the determined security policy further comprises performing one or more operations corresponding to the action specified in the request, for the determined logical resource corresponding to the logical resource identifier; and
performing one or more operations corresponding to the action specified in the request, for the determined logical resource corresponding to the logical resource identifier, further comprises:
generating a Structured Query Language (SQL) query based on the action specified in the RESTful API request and the determined logical resource;
executing the generated SQL query to produce a set of query results; and
returning the query results to the requestor from which the request was received.

2. The method of claim 1, wherein the request comprises a Hypertext Transfer Protocol (HTTP) request, and wherein the request is in compliance with a provided Representational State Transfer (RESTful) Application Program Interface (API).

3. The method of claim 2, wherein the plurality of predefined actions comprise a GET action, a PUT action, a PATCH action, a DELETE action and a POST action.

4. The method of claim 1, wherein processing the received request according to the determined security policy further comprises denying performance of the action specified in the request.

5. The method of claim 1, wherein performing one or more operations corresponding to the action specified in the request, for the determined logical resource corresponding to the logical resource identifier, further comprises:
applying one or more predicate expressions for the logical resource, based on the determined security policy; and
prior to returning the query results, filtering at least one value out of the query results, based on the retrieved one or more predicate expressions.

6. The method of claim 1, wherein the logical resource identifier comprises a Uniform Resource Identifier (URI).

7. The method of claim 1, wherein processing the received request according to the determined security policy further comprises:
determining a user role corresponding to the determined user information; and
enforcing a role-level security policy according to the determined security policy, based on the determined user role and the determined logical resource corresponding to the logical resource identifier.

8. The method of claim 1, wherein processing the received request according to the determined security policy further comprises:
determining a user identifier associated with the determined logical resource; and
enforcing a relationship security policy according to the determined security policy, based on a relationship between the user identifier associated with the determined logical resource and the user information corresponding to a requestor from which the request was received.

9. The method of claim 1, wherein determining the user information corresponding to a requestor from which the request was received further comprises:
determining the user information corresponding to a currently authenticated user on a client application from which the request was received.

10. The method of claim 9, wherein determining the user information corresponding to a requestor from which the request was received further comprises:
determining that a token object is currently assigned to the currently authenticated user; and
determining that the token object is valid.

11. A system, comprising:
a processor; and
a memory (725) containing computer program code that, when executed by the processor, performs an operation comprising:
receiving, at a server system (430), a request specifying one of a plurality of predefined actions and a logical resource identifier;
determining a logical resource uniquely identified by the logical resource identifier;
determining user information corresponding to a requestor from which the request was received;
determining a security policy to apply to the request, based on the determined user information and the logical resource;
enforcing role level security for the request, based on role(s) assigned to the determined user information,
particular logical resources may only be accessed by users assigned to certain roles, one role being a physician, and
care plans and devices of a patient are configured as different logical resources, and different logical resources can only be accessed by users assigned certain roles,
processing the received request according to the determined security policy;
processing the received request according to the determined security policy further comprises at least one of (i) denying performance of the action specified in the request, (ii) performing one or more operations corresponding to the action specified in the request, for the determined logical resource corresponding to the logical resource identifier, and (iii) performing a limited form of the one or more operations, for the determined logical resource, according to the predefined action specified in the request; and
performing one or more operations corresponding to the action specified in the request, for the determined logical resource corresponding to the logical resource identifier, further comprises:
generating a Structured Query Language (SQL) query based on the action specified in the request and the determined logical resource;
executing the generated SQL query to produce a set of query results; and
returning the query results to the requestor from which the request was received.

12. The system of claim 11, wherein the request comprises a Hypertext Transfer Protocol (HTTP) request, and wherein the request is in compliance with a provided Representational State Transfer (RESTful) Application Program Interface (API), and wherein the plurality of predefined actions comprise a GET action, a PUT action, a PATCH action, a DELETE action and a POST action.

13. The system of claim 11, wherein performing one or more operations corresponding to the action specified in the request, for the determined logical resource corresponding to the logical resource identifier, further comprises:
prior to returning the query results, filtering at least one value out of the query results, based on the determined security policy.

14. The system of claim 11, wherein processing the received request according to the determined security policy further comprises:
determining a user role corresponding to the determined user information;
enforcing a role-level security policy according to the determined security policy, based on the determined user role and the determined logical resource corresponding to the logical resource identifier;
determining a user identifier associated with the determined logical resource; and
enforcing a relationship security policy according to the determined security policy, based on a relationship between the user identifier associated with the determined logical resource and the user information corresponding to a requestor from which the request was received.

15. The system of claim 11, wherein determining the user information corresponding to a requestor from which the request was received further comprises:
determining the user information corresponding to a currently authenticated user on a client application from which the request was received; comprising:
determining that a token object is currently assigned to the currently authenticated user; and
determining that the token object is valid, upon:
determining that the token object has not expired;
determining that the token object has not been revoked at an identity server; and
determining that the token object has not been spoofed.

16. A non-transitory computer-readable medium containing computer program code that, when executed by operation of one or more computer processors, performs an operation comprising:
providing a Representational State Transfer (RESTful) Application Program Interface (API);
receiving, at a server system (430), a Hypertext Transfer Protocol (HTTP) request, formatted in compliance with the RESTful API, and specifying one of a plurality of predefined HTTP actions and a unique logical resource identifier;
determining a logical resource corresponding to the unique logical resource identifier;
determining user information corresponding to a requestor from which the HTTP request was received;
determining a security policy to apply to the HTTP request, based on the determined user information and the logical resource;
enforcing role level security for the request, based on role(s) assigned to the determined user information,
particular logical resources may only be accessed by users assigned to certain roles, one role being a physician, and
care plans and devices of a patient are configured as different logical resources, and different logical resources can only be accessed by users assigned certain roles,
determining one or more operations to perform in processing the request, based on a resource type of the logical resource and the predefined HTTP action specified in the HTTP request; and
processing the received RESTful API request by performing the one or more operations in accordance with the determined security policy,
processing the received request according to the determined security policy further comprises at least one of (i) denying performance of the action specified in the request, (ii) performing one or more operations corresponding to the action specified in the request, for the determined logical resource corresponding to the logical resource identifier, and (iii) performing a limited form of the one or more operations, for the determined logical resource, according to the predefined action specified in the request; and
performing one or more operations corresponding to the action specified in the request, for the determined logical resource corresponding to the logical resource identifier, further comprises:
generating a Structured Query Language (SQL) query based on the action specified in the request and the determined logical resource;
executing the generated SQL query to produce a set of query results; and
returning the query results to the requestor from which the request was received.

## Patentansprüche

1. Verfahren, umfassend:
Empfangen, an einem Serversystem (430), einer Anfrage, die eine von einer Mehrzahl von vordefinierten Aktionen und eine Logikressourcenkennung spezifiziert;
Bestimmen einer Logikressource, die von der Logikressourcenkennung eindeutig identifiziert wird;
Bestimmen von Benutzerinformationen, die einem Anfragesteller entsprechen, von dem die empfangene RESTfuI-API-Anfrage gekommen ist;
Bestimmen einer Sicherheitsvorschrift, die auf die Anfrage anzuwenden ist, auf Basis der bestimmten Benutzerinformationen und der Logikressource;
In-Kraft-Setzen einer Sicherheit auf Rollenebene für die Anfrage, auf Basis (von mindestens) einer Rolle, die den bestimmten Benutzerinformationen zugeordnet ist,
wobei gewisse Logikressourcen nur für Benutzer zugänglich sein können, denen bestimmte Rollen zugeordnet sind, wobei eine von den Rollen die eines Arztes ist, und
wobei Behandlungspläne und -vorrichtungen für einen Patienten als verschiedene Logikressourcen konfiguriert sind, und verschiedene Logikressourcen nur für Benutzer zugänglich sind, denen bestimmte Rollen zugeordnet sind,
Verarbeiten der empfangenen Anfrage gemäß der bestimmten Sicherheitsvorschrift;
wobei das Verarbeiten der empfangenen Anfrage gemäß der bestimmten Sicherheitsvorschrift ferner eine Durchführung einer oder mehrerer Operationen, die der in der Anfrage spezifizierten Aktion entsprechen, für die bestimmte, der Logikressourcenkennung entsprechende Logikressource umfasst; und
wobei die Durchführung einer oder mehrerer Operationen, die der in der Anfrage spezifizierten Aktion entsprechen, für die der Logikressourcenkennung entsprechende Logikressource ferner umfasst:
Erzeugen einer Abfrage in einer strukturierten Abfragesprache (SQL) auf Basis der in der RESTfuI-API-Anfrage spezifizierten Aktion und der bestimmten Logikressource;
Ausführen der erzeugten SQL-Abfrage, um einen Satz von Abfrageergebnissen zu produzieren;
und Zurückmelden der Abfrageergebnisse an den Anfragesteller, von dem die empfangene Anfrage gekommen ist.

2. Verfahren nach Anspruch 1, wobei die Anfrage eine gemäß Hypertext-Übertragungsprotokoll (HTTP) formulierte Anfrage umfasst, und wobei die Anfrage einer bereitgestellten Anwendungsprogrammschnittstelle (API) für Representational State Transfer (RESTful) entspricht.

3. Verfahren nach Anspruch 2, wobei die Mehrzahl von vordefinierten Aktionen eine GET-Aktion, eine PUT-Aktion, eine PATCH-Aktion, eine DELETE-Aktion und eine POST-Aktion umfasst.

4. Verfahren nach Anspruch 1, wobei das Verarbeiten der empfangenen Anfrage gemäß der bestimmten Sicherheitsvorschrift ferner eine Verweigerung der Durchführung der in der Anfrage spezifizierten Aktion umfasst.

5. Verfahren nach Anspruch 1, wobei das Durchführen einer oder mehrerer Operationen, die der in der Anfrage spezifizierten Aktion entsprechen, für die der Logikressourcenkennung entsprechende Logikressource ferner umfasst:
Anwenden eines oder mehrerer Prädikatausdrücke für die Logikressource auf Basis der bestimmten Sicherheitsvorschrift; und
vor dem Zurückmelden der Abfrageergebnisse: Filtern mindestens eines Wertes aus den Abfrageergebnissen auf Basis des einen oder der mehreren abgerufenen Prädikatausdrücke.

6. Verfahren nach Anspruch 1, wobei die Logikressourcenkennung eine einheitliche Ressourcenkennung (URI) umfasst.

7. Verfahren nach Anspruch 1, wobei das Verarbeiten der empfangenen Anfrage gemäß der bestimmten Sicherheitsvorschrift ferner umfasst:
Bestimmen einer Benutzerrolle, die den bestimmten Benutzerinformationen entspricht; und
In-Kraft-Setzen einer Sicherheitsvorschrift auf Rollenebene gemäß der bestimmten Sicherheitsvorschrift, auf Basis der bestimmten Benutzerrolle und der bestimmten Logikressource, die der Logikressourcenkennung entspricht.

8. Verfahren nach Anspruch 1, wobei das Verarbeiten der empfangenen Anfrage gemäß der bestimmten Sicherheitsvorschrift ferner umfasst:
Bestimmen einer Benutzerkennung, die der bestimmten Logikressource zugeordnet ist; und
In-Kraft-Setzen einer Beziehungssicherheitsvorschrift gemäß der bestimmten Sicherheitsvorschrift, auf Basis einer Beziehung zwischen der Benutzerkennung, die der bestimmten Logikressource zugeordnet ist, und den Benutzerinformationen, die einem Anfragesteller entsprechen, von dem die empfangene Anfrage gekommen ist.

9. Verfahren nach Anspruch 1, wobei das Bestimmen der Benutzerinformationen, die einem Anfragesteller entsprechen, von dem die empfangene Anfrage gekommen ist, ferner umfasst:
Bestimmen der Benutzerinformationen, die einem aktuell authentifizierten Benutzer entsprechen, an einer Client-Anwendung, aus der die Anfrage empfangen wurde.

10. Verfahren nach Anspruch 9, wobei das Bestimmen der Benutzerinformationen, die einem Anfragesteller entsprechen, von dem die empfangene Anfrage gekommen ist, ferner umfasst:
Bestimmen, dass ein Tokenobjekt aktuell dem aktuell authentifizierten Benutzer zugeordnet ist; und
Bestimmen, dass das Tokenobjekt gültig ist.

11. System, umfassend:
einen Prozessor; und
einen Speicher (725), der einen Computerprogrammcode enthält, der bei seiner Ausführung durch den Prozessor eine Operation durchführt, die umfasst:
Empfangen, an einem Serversystem (430), einer Anfrage, die eine von einer Mehrzahl von vordefinierten Aktionen und eine Logikressourcenkennung spezifiziert;
Bestimmen einer Logikressource, die von der Logikressourcenkennung eindeutig identifiziert wird;
Bestimmen von Benutzerinformationen, die einem Anfragesteller entsprechen, von dem die empfangene Anfrage gekommen ist;
Bestimmen einer Sicherheitsvorschrift, die auf die Anfrage anzuwenden ist, auf Basis der bestimmten Benutzerinformationen und der Logikressource;
In-Kraft-Setzen einer Sicherheit auf Rollenebene für die Anfrage, auf Basis (von mindestens) einer Rolle, die den bestimmten Benutzerinformationen zugeordnet ist,
wobei gewisse Logikressourcen nur für Benutzer zugänglich sein können, denen bestimmte Rollen zugeordnet sind, wobei eine von den Rollen die eines Arztes ist, und
wobei Behandlungspläne und -vorrichtungen für einen Patienten als verschiedene Logikressourcen konfiguriert sind, und verschiedene Logikressourcen nur für Benutzer zugänglich sind, denen bestimmte Rollen zugeordnet sind,
Verarbeiten der empfangenen Anfrage gemäß der bestimmten Sicherheitsvorschrift;
wobei das Verarbeiten der empfangenen Anfrage gemäß der bestimmten Sicherheitsvorschrift ferner mindestens eines umfasst von (i) Verweigern einer Durchführung der in der Anfrage spezifizierten Aktion, (ii) Durchführen einer oder mehrerer Operationen, die der in den Anfrage spezifizierten Aktion entsprechen, für die bestimmte Logikressource, die der Logikressourcenkennung entspricht, und (iii) Durchführen einer begrenzten Form der einen oder der mehreren Operationen für die bestimmte Logikressource gemäß der vorgegebenen Aktion, die in der Anfrage spezifiziert ist, und
wobei die Durchführung einer oder mehrerer Operationen, die der in der Anfrage spezifizierten Aktion entsprechen, für die der Logikressourcenkennung entsprechende Logikressource ferner umfasst:
Erzeugen einer Abfrage in einer strukturierten Abfragesprache (SQL) auf Basis der in der Anfrage spezifizierten Aktion und der bestimmten Logikressource;
Ausführen der erzeugten SQL-Abfrage, um einen Satz von Abfrageergebnissen zu produzieren; und
Zurückmelden der Abfrageergebnisse an den Anfragesteller, von dem die empfangene Anfrage gekommen ist.

12. System nach Anspruch 11, wobei die Anfrage eine gemäß Hypertext-Transferprotokoll (HTTP) formulierte Anfrage umfasst, und wobei die Anfrage einer bereitgestellten Anwendungsprogrammschnittstelle (API) für Representational State Transfer (RESTfuI) entspricht, und wobei die Mehrzahl von vordefinierten Aktionen eine GET-Aktion, eine PUT-Aktion, eine PATCH-Aktion, eine DELETE-Aktion und eine POST-Aktion umfasst.

13. System nach Anspruch 11, wobei das Durchführen einer oder mehrerer Operationen, die der in der Anfrage spezifizierten Aktion entsprechen, für die der Logikressourcenkennung entsprechende Anfrage ferner umfasst:
vor dem Zurückmelden der Abfrageergebnisse: Filtern mindestens eines Wertes aus den Abfrageergebnissen auf Basis der bestimmten Sicherheitsvorschrift.

14. System nach Anspruch 11, wobei das Verarbeiten der empfangenen Anfrage gemäß der bestimmten Sicherheitsvorschrift ferner umfasst:
Bestimmen einer Benutzerrolle, die den bestimmten Benutzerinformationen entspricht;
In-Kraft-Setzen einer Sicherheitsvorschrift auf Rollenebene gemäß der bestimmten Sicherheitsvorschrift, auf Basis der bestimmten Benutzerrolle und der bestimmten Logikressource, die der Logikressourcenkennung entspricht;
Bestimmen einer Benutzerkennung, die der bestimmten Logikressource zugeordnet ist; und
In-Kraft-Setzen einer Beziehungssicherheitsvorschrift gemäß der bestimmten Sicherheitsvorschrift, auf Basis einer Beziehung zwischen der Benutzerkennung, die der bestimmten Logikressource zugeordnet ist, und den Benutzerinformationen, die einem Anfragesteller entsprechen, von dem die empfangene Anfrage gekommen ist.

15. System nach Anspruch 11, wobei das Bestimmen der Benutzerinformationen, die einem Anfragesteller entsprechen, von dem her die empfangene Anfrage gekommen ist, ferner umfasst:
Bestimmen der Benutzerinformationen, die einem aktuell authentifizierten Benutzer an einer Client-Anwendung entsprechen, aus der die Anfrage empfangen wurde; umfassend:
Bestimmen, dass ein Tokenobjekt aktuell dem aktuell authentifizierten Benutzer zugeordnet ist; und
Bestimmen, dass das Tokenobjekt gültig ist, nach:
einer Bestimmung, dass das Tokenobjekt nicht abgelaufen ist;
einer Bestimmung, dass das Tokenobjekt nicht an einem Identitätsserver zurückgenommen wurde; und
einer Bestimmung, dass das Tokenobjekt nicht manipuliert wurde.

16. Nicht-transitorisches, computerlesbares Medium, das einen Computerprogrammcode enthält, der bei seiner Ausführung durch eine Operation eines oder mehrerer Computerprozessoren eine Operation durchführt, die umfasst:
Bereitstellen einer Anwendungsprogrammschnittstelle (API) für Representational State Transfer (RESTfuI);
Empfangen einer gemäß Hypertext-Übertragungsprotokoll (HTTP) formulierten Anfrage, die gemäß der RESTful-API formatiert ist, an einem Serversystem (430) und Spezifizieren einer von einer Mehrzahl von vordefinierten HTTP-Aktionen und einer eindeutigen Logikressourcenkennung;
Bestimmen einer Logikressource, die der Logikressourcenkennung entspricht;
Bestimmen von Benutzerinformationen, die einem Anfragesteller entsprechen, von dem die empfangene Anfrage gekommen ist;
Bestimmen einer Sicherheitsvorschrift, die auf die HTTP-Anfrage anzuwenden ist, auf Basis der bestimmten Benutzerinformationen und der Logikressource;
In-Kraft-Setzen einer Sicherheit auf Rollenebene für die Anfrage, auf Basis (von mindestens) einer Rolle, die den bestimmten Benutzerinformationen zugeordnet ist,
wobei gewisse Logikressourcen nur für Benutzer zugänglich sein können, denen bestimmte Rollen zugeordnet sind, wobei eine von den Rollen die eines Arztes ist, und
wobei Behandlungspläne und -vorrichtungen für einen Patienten als verschiedene Logikressourcen konfiguriert sind, und verschiedene Logikressourcen nur für Benutzer zugänglich sind, denen bestimmte Rollen zugeordnet sind,
Bestimmen einer oder mehrerer Operationen zur Durchführung bei der Verarbeitung der Anfrage, auf Basis eines Ressourcentyps der Logikressource und der in der HTTP-Anfrage spezifizierten, vordefinierten HTTP-Aktion; und
Verarbeiten der empfangenen RESTfuI-API-Anfrage durch Durchführen der einen oder der mehreren Operationen gemäß der bestimmten Sicherheitsvorschrift, wobei das Verarbeiten der empfangenen Anfrage gemäß der bestimmten Sicherheitsvorschrift ferner mindestens eines umfasst von (i) Verweigern einer Durchführung der in der Anfrage spezifizierten Aktion, (ii) Durchführen einer oder mehrerer Operationen, die der in den Anfrage spezifizierten Aktion entsprechen, für die bestimmte Logikressource, die der Logikressourcenkennung entspricht, und (iii) Durchführen einer begrenzten Form der einen oder der mehreren Operationen für die bestimmte Logikressource gemäß der vorgegebenen Aktion, die in der Anfrage spezifiziert ist, und
wobei die Durchführung einer oder mehrerer Operationen, die der in der Anfrage spezifizierten Aktion entsprechen, für die der Logikressourcenkennung entsprechende Logikressource ferner umfasst:
Erzeugen einer Abfrage in einer strukturierten Abfragesprache (SQL) auf Basis der in der Anfrage spezifizierten Aktion und der bestimmten Logikressource;
Ausführen der erzeugten SQL-Abfrage, um einen Satz von Abfrageergebnissen zu produzieren; und
Zurückmelden der Abfrageergebnisse an den Anfragesteller, von dem die empfangene Anfrage gekommen ist.

## Revendications

1. Procédé, comprenant :
la réception, au niveau d'un système de serveur(s) (430), d'une demande spécifiant l'une d'une pluralité d'actions prédéfinies et un identifiant de ressource logique ;
la détermination d'une ressource logique identifiée de façon unique par l'identifiant de ressource logique ;
la détermination d'une information d'utilisateur correspondant à un demandeur depuis lequel la demande RESTful APl a été reçue ;
la détermination d'une politique de sécurité à appliquer à la demande, sur la base de l'information d'utilisateur déterminée et de la ressource logique ;
la mise en application d'une sécurité du niveau des rôles pour la demande, sur la base d'un/de rôle(s) attribué(s) à l'information d'utilisateur déterminée ;
des ressources logiques particulières peuvent seulement être accédées par des utilisateurs auxquels certains rôles ont été attribués, un rôle étant une fonction de médecin ; et
les programmes et dispositifs de soins d'un patient sont configurés en tant que ressources logiques différentes et des ressources logiques différentes peuvent seulement être accédées par des utilisateurs auxquels certains rôles ont été attribués ;
le traitement de la demande reçue conformément à la politique de sécurité déterminée ;
le traitement de la demande reçue conformément à la politique de sécurité déterminée comprend en outre la réalisation d'une ou de plusieurs opérations correspondant à l'action spécifiée dans la demande, pour la ressource logique déterminée correspondant à l'identifiant de ressource logique ; et
la réalisation d'une ou de plusieurs opérations correspondant à l'action spécifiée dans la demande, pour la ressource logique déterminée correspondant à l'identifiant de ressource logique, comprend en outre :
la génération d'une requête en langage structuré de requête (SQL) sur la base de l'action spécifiée dans la demande RESTful API et de la ressource logique déterminée ;
l'exécution de la requête SQL générée pour produire un ensemble de résultats de requête ;
le retour des résultats de requête sur le demandeur depuis lequel la demande a été reçue.

2. Procédé selon la revendication 1, dans lequel la demande comprend une demande de protocole de transfert hypertexte (HTTP), et dans lequel la demande est en conformité avec une interface de programme d'application (API) de transfert d'état représentatif (RESTfuI) fournie.

3. Procédé selon la revendication 2, dans lequel les actions de la pluralité d'actions prédéfinies comprennent une action GET, une action PUT, une action PATCH, une action DELETE et une action POST.

4. Procédé selon la revendication 1, dans lequel le traitement de la demande reçue conformément à la politique de sécurité déterminée comprend en outre le rejet de la réalisation de l'action spécifiée dans la demande.

5. Procédé selon la revendication 1, dans lequel la réalisation d'une ou de plusieurs opérations correspondant à l'action spécifiée dans la demande, pour la ressource logique déterminée correspondant à l'identifiant de ressource logique, comprend en outre :
l'application d'une ou de plusieurs expressions constituées par un prédicat pour la ressource logique, sur la base de la politique de sécurité déterminée ; et
avant le retour des résultats de requête, l'évacuation par filtrage d'au moins une valeur hors des résultats de requête, sur la base des une ou plusieurs expressions constituées par un prédicat extraites.

6. Procédé selon la revendication 1, dans lequel l'identifiant de ressource logique comprend un identifiant de ressource uniforme (URI).

7. Procédé selon la revendication 1, dans lequel le traitement de la demande reçue conformément à la politique de sécurité déterminée comprend en outre :
la détermination d'un rôle d'utilisateur correspondant à l'information d'utilisateur déterminée ; et
la mise en application d'une politique de sécurité du niveau des rôles conformément à la politique de sécurité déterminée, sur la base du rôle d'utilisateur déterminé et de la ressource logique déterminée correspondant à l'identifiant de ressource logique.

8. Procédé selon la revendication 1, dans lequel le traitement de la demande reçue conformément à la politique de sécurité déterminée comprend en outre :
la détermination d'un identifiant d'utilisateur associé à la ressource logique déterminée ; et
la mise en application d'une politique de sécurité relationnelle conformément à la politique de sécurité déterminée, sur la base d'une relation entre l'identifiant d'utilisateur associé à la ressource logique déterminée et l'information d'utilisateur correspondant à un demandeur depuis lequel la demande a été reçue.

9. Procédé selon la revendication 1, dans lequel la détermination de l'information d'utilisateur correspondant à un demandeur depuis lequel la demande a été reçue comprend en outre :
la détermination de l'information d'utilisateur correspondant à un utilisateur présentement authentifié sur une application de client depuis laquelle la demande a été reçue.

10. Procédé selon la revendication 9, dans lequel la détermination de l'information d'utilisateur correspondant à un demandeur depuis lequel la demande a été reçue comprend en outre :
la détermination du fait qu'un objet jeton est présentement attribué à l'utilisateur présentement authentifié ; et
la détermination du fait que l'objet jeton est valide.

11. Système, comprenant :
un processeur ; et
une mémoire (725) qui contient un code de programme informatique qui, lorsqu'il est exécuté par le processeur, réalise une opération comprenant :
la réception, au niveau d'un système de serveur(s) (430), d'une demande spécifiant l'une d'une pluralité d'actions prédéfinies et un identifiant de ressource logique ;
la détermination d'une ressource logique identifiée de façon unique par l'identifiant de ressource logique ;
la détermination d'une information d'utilisateur correspondant à un demandeur depuis lequel la demande a été reçue ;
la détermination d'une politique de sécurité à appliquer à la demande, sur la base de l'information d'utilisateur déterminée et de la ressource logique ;
la mise en application d'une sécurité du niveau des rôles pour la demande, sur la base d'un/de rôle(s) attribué(s) à l'information d'utilisateur déterminée ;
des ressources logiques particulières peuvent seulement être accédées par des utilisateurs auxquels certains rôles ont été attribués, un rôle étant une fonction de médecin ; et
les programmes et dispositifs de soins d'un patient sont configurés en tant que ressources logiques différentes et des ressources logiques différentes peuvent seulement être accédées par des utilisateurs auxquels certains rôles ont été attribués ;
le traitement de la demande reçue conformément à la politique de sécurité déterminée ;
le traitement de la demande reçue conformément à la politique de sécurité déterminée comprend en outre la réalisation d'au moins une opération parmi (i) le rejet de la réalisation de l'action spécifiée dans la demande, (ii) la réalisation d'une ou de plusieurs opérations correspondant à l'action spécifiée dans la demande, pour la ressource logique déterminée correspondant à l'identifiant de ressource logique et (iii) la réalisation d'une forme limitée des une ou plusieurs opérations, pour la ressource logique déterminée, conformément à l'action prédéfinie spécifiée dans la demande ; et
la réalisation d'une ou de plusieurs opérations correspondant à l'action spécifiée dans la demande, pour la ressource logique déterminée correspondant à l'identifiant de ressource logique, comprend en outre :
la génération d'une requête en langage structuré de requête (SQL) sur la base de l'action spécifiée dans la demande et de la ressource logique déterminée ;
l'exécution de la requête SQL générée pour produire un ensemble de résultats de requête ; et
le retour des résultats de requête sur le demandeur depuis lequel la demande a été reçue.

12. Système selon la revendication 11, dans lequel la demande comprend une demande de protocole de transfert hypertexte (HTTP), et dans lequel la demande est en conformité avec une interface de programme d'application (API) de transfert d'état représentatif (RESTfuI) fournie, et dans lequel les actions de la pluralité d'actions prédéfinies comprennent une action GET, une action PUT, une action PATCH, une action DELETE et une action POST.

13. Système selon la revendication 11, dans lequel la réalisation d'une ou de plusieurs opérations correspondant à l'action spécifiée dans la demande, pour la ressource logique déterminée correspondant à l'identifiant de ressource logique, comprend en outre :
avant le retour des résultats de requête, l'évacuation par filtrage d'au moins une valeur hors des résultats de requête, sur la base de la politique de sécurité déterminée.

14. Système selon la revendication 11, dans lequel le traitement de la demande reçue conformément à la politique de sécurité déterminée comprend en outre :
la détermination d'un rôle d'utilisateur correspondant à l'information d'utilisateur déterminée ;
la mise en application d'une politique de sécurité du niveau des rôles conformément à la politique de sécurité déterminée, sur la base du rôle d'utilisateur déterminé et de la ressource logique déterminée correspondant à l'identifiant de ressource logique ;
la détermination d'un identifiant d'utilisateur associé à la ressource logique déterminée ; et
la mise en application d'une politique de sécurité relationnelle conformément à la politique de sécurité déterminée, sur la base d'une relation entre l'identifiant d'utilisateur associé à la ressource logique déterminée et l'information d'utilisateur correspondant à un demandeur depuis lequel la demande a été reçue.

15. Système selon la revendication 11, dans lequel la détermination de l'information d'utilisateur correspondant à un demandeur depuis lequel la demande a été reçue comprend en outre :
la détermination de l'information d'utilisateur correspondant à un utilisateur présentement authentifié sur une application de client depuis laquelle la demande a été reçue ; comprenant :
la détermination du fait qu'un objet jeton est présentement attribué à l'utilisateur présentement authentifié ; et
la détermination du fait que l'objet jeton est valide, sur la base de :
la détermination du fait que l'objet jeton n'a pas expiré ;
la détermination du fait que l'objet jeton n'a pas été révoqué au niveau d'un serveur d'identité(s) ; et
la détermination du fait que l'objet jeton n'a pas été usurpé.

16. Support lisible par ordinateur non transitoire contenant un code de programme informatique qui, lorsqu'il est exécuté au moyen de l'activation d'un ou de plusieurs processeurs informatiques, réalise une opération comprenant :
la fourniture d'une interface de programme d'application (API) de transfert d'état représentatif (RESTfuI) ;
la réception, au niveau d'un système de serveur(s) (430), d'une demande de protocole de transfert hypertexte (HTTP), formatée conformément à la RESTfuI API et spécifiant l'une d'une pluralité d'actions HTTP prédéfinies et un identifiant de ressource logique unique ;
la détermination d'une ressource logique correspondant à l'identifiant de ressource logique unique ;
la détermination d'une information d'utilisateur correspondant à un demandeur depuis lequel la demande HTTP a été reçue ;
la détermination d'une politique de sécurité à appliquer à la demande HTTP, sur la base de l'information d'utilisateur déterminée et de la ressource logique ;
la mise en application d'une sécurité du niveau des rôles pour la demande, sur la base d'un/de rôle(s) attribué(s) à l'information d'utilisateur déterminée ;
des ressources logiques particulières peuvent seulement être accédées par des utilisateurs auxquels certains rôles ont été attribués, un rôle étant une fonction de médecin ; et
les programmes et dispositifs de soins d'un patient sont configurés en tant que ressources logiques différentes et des ressources logiques différentes peuvent seulement être accédées par des utilisateurs auxquels certains rôles ont été attribués ;
la détermination d'une ou de plusieurs opérations à réaliser lors du traitement de la demande, sur la base d'un type de ressource de la ressource logique et de l'action HTTP prédéfinie spécifiée dans la demande HTTP ; et
le traitement de la demande RESTful API reçue en réalisant les une ou plusieurs opérations conformément à la politique de sécurité déterminée ; le traitement de la demande reçue conformément à la politique de sécurité déterminée comprend en outre la réalisation d'au moins une opération parmi (i) le rejet de la réalisation de l'action spécifiée dans la demande, (ii) la réalisation d'une ou de plusieurs opérations correspondant à l'action spécifiée dans la demande, pour la ressource logique déterminée correspondant à l'identifiant de ressource logique et (iii) la réalisation d'une forme limitée des une ou plusieurs opérations, pour la ressource logique déterminée, conformément à l'action prédéfinie spécifiée dans la demande ; et
la réalisation d'une ou de plusieurs opérations correspondant à l'action spécifiée dans la demande, pour la ressource logique déterminée correspondant à l'identifiant de ressource logique, comprend en outre :
la génération d'une requête en langage structuré de requête (SQL) sur la base de l'action spécifiée dans la demande et de la ressource logique déterminée ;
l'exécution de la requête SQL générée pour produire un ensemble de résultats de requête ; et
le retour des résultats de requête sur le demandeur depuis lequel la demande a été reçue.
